# EUROPEAN PATENT SPECIFICATION

(11) **EP 1 632 212 B1**
(45) Date of publication and mention of the grant of the patent: **16.04.2008**
(21) Application number: 05017500.9
(22) Date of filing: 11.08.2005
(51) Int. Cl.: A61K 8/04, A61K 8/19, A61K 8/87, A61K 8/81, A61Q 1/00, A61Q 1/02, A61Q 1/10, C09C 1/00, C09C 3/00

(54) **Scale-like composite particles and cosmetics with the same blended therein**
Plättchenförmige Komposit-Partikeln und Kosmetika enthaltend die selben
Particules lamellaires composites et compositions cosmetiques les contenant

(30) Priority: 11.08.2004 JP 2004234396
(43) Date of publication of application: 08.03.2006
(73) Proprietor: CATALYSTS & CHEMICALS INDUSTRIES CO., LTD., Kawasaki-shi Kanagawa (JP)
(72) Inventor: Miyazaki, Takumi Catalysts & Chemicals Ind. Co Ltd, Wakamatsu-ku Kitakyushu-shi Fukuoka (JP); Tanaka, Hirokazu Catalysts & Chemicals Ind. Co Ltd, Wakamatsu-ku Kitakyushu-shi Fukuoka (JP); Matsumoto, Shuji Catalysts & Chem. Ind. Co., Ltd., Wakamatsu-ku Kitakyushu-shi Fukuoka (JP)
(74) Representative: HOFFMANN EITLE

(56) References cited:
- EP-A- 1 338 627
- EP-A- 1 426 031
- US-A- 4 772 331
- US-A- 5 037 475

## Description

### BACKGROUND OF THE INVENTION AND RELATED ART STATEMENT

The present invention relates to novel scale-like composite particles and cosmetics with the same blended therein.

The scale-like inorganic particles such as mica, talc, and sericite are blended in cosmetics for making up such as powder foundation. The effects obtained by blending the scale-like inorganic particles in the cosmetics include the excellent spreading characteristics, improved dispersibility of coloring pigments on human skin, and high adhesiveness to skin, all of which are indispensable in the cosmetics for making up.
Most of the scale-like inorganic particles are made of inorganic oxides such as silica, alumina, and magnesium oxide, and give hard feel, and therefore recently there have been made various efforts for improving the feel to provide the feelings such as lightness, smoothness, and softness by making use of flaks of the inorganic oxides.

Japanese Patent Publication No. HEI 7-103324 (Patent document 1) discloses a soft matrix made from small flakes prepared by containing minute spherical particles in a small flake matrix such as mica without fixing the particles to the matrix, and in this matrix, even when the small flakes aggregate with each other, the aggregates can easily be separated and dispersed.
Further the present inventors have proposed use of flaky fine powder coated with spherical silica particles which can suppress excessive glazing and ensure soft focusing effect and also provides slippery characteristic and improved feel in use (Japanese Patent No. 2784261: Patent document 2).

Although these powder materials provide the excellent slippery characteristic, when the materials are extended again and again on human skin, sometimes the spherical particles get separated from the flaky powders with the ball bearing effect lost, so that there is the problem in preservation of the slippery characteristic for a long period of time. Further, even when the powders are blended in cosmetics and applied on human skin, the oily feel felt when an oily material is applied on human skin, can not be provided.

Further, the present inventors have proposed scale-like inorganic particles prepared by coating scale-like inorganic particles with resin having 100% modulus in the tensile test of 50 to 3000 N/cm² and capable of ensuring soft feel, excellent adhesiveness to human skin for following movement of human skin, and the effect of suppressing excessive glazing (Japanese Patent Laid-Open Publication No, 2002-69329: Patent document 3).
However, the scale-like inorganic particles have the problem that the particles give the bulky feel and are not homogeneously extended, when applied on human skin, because of the characteristics of the resin used for coating, so that the oily feel can not be obtained.

- [Patent document 1]: Japanese Patent Publication No. HEI 7-103324; US 5407746
- [Patent document 2]: Japanese Patent No. 2784261; EP 0543999B1
- [Patent document 3]: Japanese Patent Laid-Open Publication No. 2002-69329; US 6825259 B2

US 4,772,331 relates to free flowing coloured flaky pigments which are obtained by uniformly adhering a finely divided colour pigment onto a flaky substrate such as talc, kaolin or mica by the use of an high molecular weight organic compound as a binder. The colour pigment is finely divided by a sand mill, ball mill or roll mill.

US 5,037,475 discloses a primary colored metallic pigment comprising a metallic pigment in the form of flaky grain, a coloring pigment and a thermally polymerized carboxylic acid, the coloring pigment being chemically adsorbed on the surface of the metallic pigment via the thermally polymerized carboxylic acid. This document pertains to non-cosmetic applications, such as bright paint, bright ink and bright plastics which contain the colored metallic pigment. The coloring pigment can be organic or inorganic, the latter including various oxide pigments.

EP 1 426 031 A1 which is in the name of the present applicant discloses modified inorganic fine particles for cosmetics having on a surface thereof a polymer layer prepared by plasma-polymerizing specific monomers. According to one embodiment of this polymer layer, the same bonds a functional organic compound which is preferably selected from dyes, UV absorbers and skin-chapping inhibitors. According to this embodiment, the functional organic compounds are thus chemically bound to the polymer layer. Alternatively, they are physically fixed entwining with the mesh of the polymer layer.

### SUMMARY OF THE INVENTION

The present invention was made to overcome the problems described above, and an object of the present invention is to provide scale-like composite particles having the so-called oily feel, soft and smooth feel, adaptability to application, excellent spreading characteristic. Another object of the present invention is to provide smooth and soft cosmetics with the scale-like composite particles blended therein which can provide the oily feel when applied on human skin, and also which can preserve the moisty and soft feels like those of real human skin even after application thereon to skin.

The scale-like composite particles according to the present invention include resin coating the surface and further include spherical fine particles with the average particle diameter in the range from 0.1 to 3.0 µm adhered on the resin surface.
The spherical fine particles are inorganic oxide particles, and also preferably form a single layer in the adhered state.
A quantity of the spherical fine particles is preferably in the range from 1/99 to 60/40 as expressed by a weight ratio of spherical fine particles versus scale-like inorganic particles coated with resin.

A quantity of resin coating the resin-coated scale-like inorganic particles is preferably in the range from 0.1/99.9 to 20/80 as expressed by a weight ratio of resin versus scale-like inorganic particles.
The resin-coated scale-like inorganic particles preferably have the average particle diameter (Df) in the range from 1 to 50 µm, the average thickness (Tf) in the range from 0.01 to 1 µm, and the ratio (Df/Tf) of average diameter (Df) versus average thickness (Tf) in the range from 10 to 300.
The 100% modulus of the resin in the tensile test is preferably in the range from 50 to 3000 N/cm².

The resin is preferably one or more selected from polyurethane, styrene-butadiene copolymer, acrylonitrile-butadiene copolymer, silicone-based elastomer, and polyolefin-based elastomer.
The cosmetics according to the present invention includes any of the scale-like composite particles described above blended therein.

The scale-like composite particles according to the present invention provide, when applied on human skin, in addition to particular oily feel, soft and smooth feel. Further the smoothness is preserved for a longer period of time as compared to other known fine particles blended in cosmetics, and also the slippery characteristics is preserved even after the particles are blended in cosmetics and applied on human skin.

### DETAILED DESCRIPTION OF THE EMBODIMENTS

Preferred embodiments of the present invention are described below.

### Scale-like composite particles

The scale-like composite particles according to the present invention includes resin coating surface thereof and spherical fine particles adhered on the resin.

### [Scale-like inorganic particles]

The scale-like inorganic particles used in the present invention include natural minerals such as mica, talc, sericite; synthetic mica, synthetic sericite, plate-like titanium oxide, plate-like silica, plate-like aluminum, boron nitride, barium sulfate, plate-like titania silica composites, and bismuth oxychloride. Further it is possible to use in the present invention scale-like composite particles including any of the scale-like inorganic particles described above as a matrix and one or more inorganic oxides selected from the group consisting of titanium oxide, aluminum oxide, ferric oxide, silica, cerium oxide, and zirconium oxide. Further the scale-like inorganic particles carrying organic dyes and/or pigments thereon may be used in the present invention. In the present invention, a single type of scale-like inorganic particles may be used, but also a combination of two or more types of scale-like inorganic particles may be used.

Generally an average diameter of the scale-like inorganic particles as described above is preferably in the range from 1 to 50 µm, and more preferably in the range from 5 to 30 µm. Furthier the thickness of the scale-like inorganic particles is preferably in the range from 0.01 to 1 µm, and more preferably in the range from 0.1 to 0.5 µm.
When the average diameter of the scale-like inorganic particles is less than 1 µ m, such characteristics of the scale-like inorganic particles as the spreading characteristics and improved dispersibility may be lost. When the average diameter of the scale-like inorganic particles is over 50 µm, glittering due to the excessive glazing may disadvantageously be provided.

The average diameters of the scale-like inorganic particles, resin-coated scale-like inorganic particles described hereinafter, and scale-like composite particles as used herein indicates an average value of a major axis and a minor axis of each of the scale-like particles viewed in the direction orthogonal to a surface of the scale-like particles, and can be obtained by photographing the scale-like particles with a scanning electron microscope (SEM), and measuring a major axis and a minor axis of each of 100 particles.

### [Resin]

There is no specific restriction over the resin as described above, and any known resin may be used for this purpose.
Especially, it is advantageous to use resin having elasticity like that of rubber such as polyurethane, styrene-butadiene copolymer, acrylonitrile-butadiene copolymer, silicone rubber, natural rubber, nylon-based elastomer, polyester-based elastomer, polyolefin-based elastomer.

Further the resin may has a functional group such as carboxylic acid, sulfonic acid, amine and derivatives thereof for forming emulsion, and also may have any group for bridging such as, for instance, an epoxy group, a carbodiimide group, and a silanol group.
Generally, these resins are positively charged when static electricity is generated. The spherical fine particles described hereinafter are made from inorganic oxides such as silica, leading to fine particles being negatively charged, so that adhesion between the resin and the inorganic particles easily occurs, and spherical fine particles hardly get separated from the resin-coated scale-like inorganic particles as the matrix due to the shearing stress generated when the scale-like composite particles are applied on human skin, and the slippery characteristics due to the ball bearing effect is preserved for a long period of time.

The resin has the 100% modulus in the tensile test preferably in the range from 50 to 3000 N/cm² and more preferably in the range from 50 to 1500 N/cm². When the 100% modulus of the resin in the tensile test is less than 50 N/cm², the adhesiveness of the obtained scale-like composite particles, and the particles may cause blocking with each other to form an aggregate, which is not preferable from the view point of the feel such as the spreading characteristic. When the 100% modulus of the resin in the tensile test is more than 3000 N/cm², softness of the resin becomes lower, and the effect of providing the oily feel becomes insufficient. Namely the oily feel expressed in the scale-like composite particles is combined with the softness of the resin layer as well as with the ball bearing effect with the spherical fine particles on a surface of the resin layer to provide the oily feel felt with senses of a human body.

Measurement of the 100% modulus in the tensile test is as described below.
At first, when the resin is an emulsion, the resin is used as it is. Otherwise, the resin is changed to a solution with an organic solvent best suited to the purpose. Then the resin emulsion or resin solution is applied with the doctor blade method and dried to form a film with the thickness of 30 µm. Then the film is punched out with an H-shaped form to provide a film for testing. The left and right edge sections of the H-shaped film for testing are pulled at the tensile speed of 20 mm/minute to obtain a relation between the extension (cm) and a stress (applied load(N)/cross section (cm²)). The "100% modulus" is defined as a stress (N/cm²) when the length of the tested film becomes twice of the original length.

A quantity of resin used for coating the resin-coated scale-like inorganic particles expressed as a weight ratio of resin versus scale-like inorganic particles is preferably in the range from 0.1/99.9 to 20/80, and more preferably in the range from 0.5/99.5 to 5/95.
When a quantity of resin as expressed as a weight ratio of resin versus scale-like inorganic particles is less than 0.1/99.9, the quantity of resin is too small, and sometimes the coating effect of providing the soft feel may be insufficient. When the quantity of resin as expressed as a weight ratio of resin versus scale-like inorganic particles is over 20/80, although depending on a type of the resin used, blocking between particles easily occurs, which is not preferable.

Surfaces of the scale-like inorganic particles are preferably coated with the resin homogeneously. When the coating state is not homogeneous, the effect of providing the soft feel is not sufficient with the oily feel lost, and also the spreading characteristic becomes remarkably lower.

### [Resin-coated scale-like inorganic particles]

An average particle diameter (Df) of the scale-like inorganic particles coated with the resin is preferably in the range from 1 to 50 µm, an average thickness (Tf) thereof is preferably in the range from 0.01 to 1 µm, and a ratio (Df/Tf) of average particle diameter (Df) versus average thickness (Tf) is preferably in the range from 10 to 300. When the average particle diameter (Df) of the scale-like inorganic particles coated with the resin is less than 1 µm, the excellent spreading characteristic, improved dispersibility and other excellent characteristics of the scale-like inorganic particles may be lost, and when the average particle diameter (Df) is over 50 µm, the glittering due to the excessive glazing is generated, which is not preferable. When the average thickness (Tf) is less than 0.01 µm, the particles may easily be broken during the dispersing process in preparation of cosmetics, while, when the average thickness (Tf) is over 1 µm, the excellent spreading characteristics and adhesiveness of the scale-like particles becomes lower, which is not preferable.

### [Method of producing the resin-coated scale-like inorganic particles]

There is no specific restriction over the method of producing the resin-coated scale-like inorganic particles according to the present invention, and any known method may be employed so long as surface of the scale-like inorganic particles can homogeneously be coated with resin. For instance, the following method may advantageously be employed.

(1) A method in which scale-like inorganic particles are dispersed in resin emulsion or latex, stability in dispersion of the resin composition particles is lowered by adjusting pH of the dispersion liquid, the resin composition particles are deposited and laminated on surfaces of the scale-like inorganic particles, and then are dried. A range of pH varies according to a type of the resin emulsion or latex used in the step, but any pH value may be allowable so long as the stability in dispersion of the resin composition particles can be lowered. Further, a dispersion liquid prepared by dispersing granulized powder of resin in water or alcohol may be employed in the resin emulsion or latex, and further the resin may be used as a solution prepared by dissolving the resin in a solvent such as water, toluene, methylethyl ketone, and xylene. The resin may further be emulsion or latex of self emulsification type prepared by dispersing the resin in water or of compulsory emulsification type prepared with an emulsifying agent. Further the resin may be used as a solution prepared by dissolving the resin in a solvent.

(2) A method in which scale-like inorganic particles are dispersed in resin emulsion or latex and dried by spray drying or the like.
(3) A method in which the scale-like inorganic particles are dispersed in a resin monomer solution or dispersion liquid to polymerize the resin monomers and then the resin is coated on the scale-like inorganic particles.

(4) A method in which granulized resin powder and scale-like inorganic particles are mixed with each other and a physical force is loaded to the mixture to soften and melt the resin composition with frictional heat generated in the step for coating the melted resin on the scale-like inorganic particles. As a device for mixing and adhering the resin, for instance, the Mechano-Fusion System produced by Hosokawa Micron (K. K.) may advantageously be used.
Also after the scale-like inorganic particles are coated with the resin, the resin-coated scale-like inorganic particles may be heated for adhering the resin on the scale-like inorganic particles for use.

### [Spherical fine particles made from inorganic oxide]

As the spherical fine particles used in the present invention, fine particles of inorganic oxide or composite inorganic oxide including one or more selected from silica, titanium oxide, alumina, zirconium oxide, ferric oxide, zinc oxide, and cerium oxide may advantageously be used. In this step, when such materials as silica, titanium oxide, alumina, and zirconium oxide are used, it is easy to obtain the spherical particles, and the cosmetics with the scale-like inorganic particles including the particles of the material adhered thereon can provide the soft feel. Also it is advantageous to use, for instance, the particles prepared by mixing ferric oxide excellent in the coloring characteristic or cerium oxide excellent in the capability of shuttering UV rays in such a material as silica, titanium oxide, alumina, and zirconium oxide. Further the different types of spherical fine particles may be mixed in use.

Further in addition to the inorganic fine particles described above, such organic spherical particles as nylon, silicone resin, polymethyl methacrylate, or polystyrene may be used in the present invention.
An average particle diameter of the spherical fine particles is preferably in the range from 0.1 to 3.0 µm, and more preferably in the range from 0.2 to 1.5 µm.

Then the average particle diameter of the spherical fine particles is less than 0.1 µm, the oily feel can not be obtained, and the smoothness is low. When the average particle diameter of the spherical fine particles is over 3 µm, it is difficult to have the spherical particles adhered on the resin-coated scale-like inorganic particles, and even if it is possible, the oily and smooth feel and the spreading characteristic may be insufficient.
Further distribution of diameters of the spherical fine particles is preferably homogeneous. The closer the distribution range of the diameters is, the higher bearing effect can be obtained. On the contrary, when the distribution range is wide, the oily feel drops.

A quantity of adhered spherical fine particles varies according to size of the spherical fine particles, and the quantity as expressed by a weight ratio of spherical fine particles versus resin-coated scale-like inorganic particles is preferably in the range from 1/99 to 60/40, and more preferably in the range from 3/97 to 50/50. The quantity of adhered spherical fine particles may be selected according to an average diameter of the spherical fine particles, and should preferably be sufficient for homogeneously coating surfaces of the resin-coated scale-like inorganic particles with a single layer. When the quantity is excessive, the oily feel may be spoiled.

For instance, when spherical silica particles with the average particle diameter of 0.6 µm is adhered on mica with the average particle diameter of 13 µm and the average thickness of 0.25 µm and coated by polyurethane by 2 weight %, the weight ratio of less than 1/99 is insufficient for providing the oily feel, and generally when the quantity is in the range from 20/80 to 40/60 sufficient for forming a single layer of the spherical fine particles, the oily feel can be obtained. When the weight ratio is over 50/50, the oily and smooth feel becomes lower with the spreading characteristic worsened.

When spherical silica particles with the average particle diameter of 1.0 µm are adhered polyurethane-coated mica in the same way, if the weight ratio is less than 1/99, the effect of providing the oily feel is lost almost completely. When the weight ratio is in the range from 30/70 to 50/50, the very oily feel is obtained, and when the weight ratio is over 60/40, the spreading characteristic lowers.

### [Method for adhering spherical particles]

There is no specific restriction over the method for adhering spherical particles on the resin-coated scale-like inorganic particles so long as scale-like composite particles having, smooth feel, excellent applicability, spreading characteristic and the like, and for instance the following method may be employed.

(1) A method in which the resin-coated scale-like inorganic particles and spherical fine particles are dispersed in water, alcohol, or a mixture dispersant thereof, and the dispersion liquid is sprayed in a heated air stream for drying.
In this method, generally a total concentration of the resin-coated scale-like inorganic particles and spherical particles in the dispersion liquid is preferably in the range from 5 to 50 weight %, and more preferably in the range from 10 to 30 weight %. Temperature of the heated air is generally in the range from 60 to 130 °C.

(2) A method in which the resin-coated scale-like inorganic particles and spherical fine particles are put in a container of a mixer, a physical force is loaded, for instance, by rotating the container so that static electricity is generated due to friction between particles, and then the spherical particles are adhered on the resin-coated scale-like inorganic particles. As a device used in this process, for instance, Mechano Fusion System produced by Hosokawa Micron (K. K.) may advantageously be employed.
Further, when the spherical fine particles are adhered on the resin-coated scale-like inorganic particles and the obtained scale-like inorganic particles aggregate, the aggregate may be crushed for use according to the necessity.

### Cosmetics

The cosmetics according to the present invention is described below.
The cosmetics according to the present invention includes the scale-like composite particles described above blended therein together with various ingredients described below. A blending ratio of the scale-like composite particles in the cosmetics is preferably in the range from 1 to 90 weight %, and more preferably in the range from 3 to 40 weight %. When the blending ratio is less than 1 weight %, the soft and smooth feel can not be obtained, and when the blending ratio is over 90 weight %, the coloring characteristic, decorative characteristic, the oily feel, and the like generally required for cosmetics are lost.

The ingredients to be blended in the cosmetics according to the present invention include, for instance, lipids such as olive oil, canola oil, beef tallow; waxes such as jojoba oil, canderilla wax, yellow beeswax; carbon hydrides such as paraffin, sukuwaran, synthetic and botanical sukuwaran, α-olefin oligomer, microcrystalline wax; fatty acids such as stearic acid, myristic acid, oleic acid, α-hydroxylic acid, linoleic acid, lactic acid; alcohols such as isostearyl alcohol, octydedecanol, lauryl alcohol, ethanol, isopropanol, butyl alcohol, myristic alcohol, cetanole, stearyl alcohol, befenyl alcohol; alkylglycer ethers; esters such as myristic acid isopropyl, palmitic acid isopropyl, stearic acid ethyl, oleic acid ethyl, lauric acid cetyl, and oleic acid decyl; polyalcohols such as ethylene glycol, triethylene glycol, polyethylene glycol, propylene glycol, butylene glycol, glycerin, diglycerin; sugars such as sorbitol, glucose, sucrose, trehalose, pullulan; ascorbic acid derivatives such as arbutin, vitamin C, ascorbic acid sodium; circular dimethyl silicone oil such as carotenoid, flavonoid, tannin, lignan, saponin, retinoic acid and retinoic acid structural homologues, methylpolysiloxane, methylhydrogen polysiloxane, methylphenyl silicone oil, decamethyl cyclopentasiloxane; silicone oils such as various types of denatured silicone oils; fluorine oils such as perfluoropolyether; polymer molecules such as Arabian rubber, carrageenan, agar, xanthan rubber, gelatin, alginic acid, Cyamoposis Gum, albumin, pullulan, carboxyvinyl polymer, cellulose and derivatives thereof, polyacrylic amide, polyacrylic acid sodium, polyvinyl alcohol; anion-, cation-, or non-ionic surfactants; extracted materials from animals or plants; amino acids and peptides; vitamins; UV-ray protective agents; antibacterial and antiseptic agents; antioxidants; denatured or not-denatured clay minerals; solvents such as butyl acetate, acetone, and toluene; particles of inorganic oxides and/or hydroxides having various diameters, diameter distributions, and particle forms and also containing one or more of titanium oxide, zinc oxide, aluminum oxide, aluminum hydroxide, colcothar, yellow ferric oxide, black ferric oxide, cerium oxide, zirconium oxide, silica and the like; organic powder of polymethyl methacrylate, nylon, silicone resin, silicone rubber, polyethylene, polyester, or polyurethane and having various diameters, diameter distributions, and particle forms; color materials such as ultramarine blue pigment, iron blue pigment, manganese violet, chromium hydroxide, tar pigment, natural coloring material; one of more of materials such as mica titanium, mica, talc, sericite, boron nitride, barium sulfate, synthetic mica, synthetic sericite, plate-like titanium oxide, plate-like silica, plate-like aluminum oxide, and composite materials of the materials with inorganic compounds or organic compounds; water; and aroma chemicals and the like.

Various ingredients blended in the cosmetics according to the present invention are not limited to the materials described above, and any of raw materials listed on The Japanese Cosmetic Ingredient Codex (published by Yakuji Nippou Ltd.) and The Japanese Cosmetic Ingredient Codex (Audited by Health and Welfare Ministry, Pharmaceutical Affairs Dep., Examination Dev., published by Yakuji Nippou Ltd.) may be included in the cosmetics according to the present invention. Of these ingredients, those having the powder form may be used as they are, or may be subjected to surface processing with silicone, fluorine, metallic soap, lauroyl lycine, and various types of oil solutions or used to form a complex. Further the ingredients may be used as dispersing elements.

The cosmetics according to the present invention may be produced by any known method, and may be used in various forms such as powder, liquid, cream, and the like including foundation, eye shadow, loose powder, liquid foundation, cream, emulsion, lotion, lipsticks and the like.
When producing cosmetics, the scale-like composite particles may be subjected to surface processing with silicone, fluorine, metallic soap, lauroyl lycine, and various types of oil solutions for use.

### EXAMPLES

The present invention is now described further specifically with reference to the following examples.

### [Example 1]

As resin, 100 g of a water dispersion solution of a non-yellowing type of polyurethane resin (manufactured by MITSUI TAKEDA CHEMICAL K.K.: glass transition temperature; 65°C, 100% modulus in the tensile test; 500 N/cm², resin particle diameter; 0.07 µm, self-emulsifying type, solid content concentration; 30% by weight) and, as scale-like inorganic particles, 1470 g of mica having an average particle diameter of 13 µ m and a thickness of 0.25 µm were mixed in 5930 g of deionized water to prepare a mixture solution having a weight ratio of the polyurethane resin to mica of 2/98 and a solid content concentration of 20 % by weight. The mixture solution was dried by being sprayed in a dry atmosphere at a temperature of 70°C and at a humidity of 5%, and was heated at 80°C for 24 hours to obtain resin-coated scale-like inorganic particles (A). When this resin-coated scale-like inorganic particles (A) was applied to skin, softer feel and higher adhesiveness to skin was provided with glazing being suppressed, as compared to mica alone applied to skin, while in turn, a little bulky feel was given, smoothness was not sufficient when applied on skin, and homogeneous application was difficult.

400 g of resin-coated scale-like inorganic particles (A) was added to and mixed with 1600 g of deionized water, and 244 g of a spherical silica dispersion solution having an average particle diameter of 0.55 µm (manufactured by Catalysts & Chemicals Industries Co., Ltd. (CCIC): SS-550, solid content concentration; 18%) was further mixed therewith. The weight ratio of the spherical silica and resin-coated scale-like inorganic particles (A) at that time was 10/90. The dispersion solution was dried by being sprayed in a dry atmosphere at a temperature of 70°C and at a humidity of 5%, and was collected to a vessel cooled to 10°C to obtain scale-like composite particles (A-1) with spherical silica particles adhered thereto.
When the scale-like composite particles (A-1) was applied to skin, oily feel was given, and smooth and homogeneous application was possible, and a touch after the application also kept oily feel and slippery characteristic.

### [Example 2]

300 g of resin-coated scale-like inorganic particles (A) obtained in the same way as Example 1 was added to and mixed with 1200 g of deionized water, and 1111 g of a spherical silica dispersion solution having an average particle diameter of 0.55 µm (manufactured by CCIC: SS-550, solid content concentration; 18%) was further mixed therewith. The weight ratio of the spherical silica and resin-coated scale-like inorganic particles (A) at that time was 40/60.

The dispersion solution was spray-dried in the same way as Example 1 to obtain scale-like composite particles (A-2) with spherical silica particles adhered thereto.
When the scale-like composite particles (A-2) was applied to skin, highly oily feel was given, and a touch after the application provides oily feel and slippery characteristic.

### [Example 3]

300 g of resin-coated scale-like inorganic particles (A) obtained in the same way as Example 1 was added to and mixed with 1200 g of deionized water, and 375 g of a spherical silica dispersion solution having an average particle diameter of 0.3 µm (manufactured by CCIC: SS-300, solid content concentration; 20%) was further mixed therewith. The weight ratio of the spherical silica and resin-coated scale-like inorganic particles (A) at that time was 20/80.

The dispersion solution was spray-dried in the same way as Example 1 to obtain scale-like composite particles (A-3) with spherical silica particles adhered thereto.
When the scale-like composite particles (A-3) was applied to skin, oily, smooth and homogeneous application characteristic was given, and a touch after the application provides oily feel and slippery characteristic.

### [Example 4]

As resin, 100 g of styrene butadiene-based resin latex (manufactured by NIHON ZEON K.K.: glass transition temperature; 12°C, 100% modulus in the tensile test; 620 N/cm², resin particle diameter; 0.15 µm, solid content concentration; 49% by weight) and, as scale-like inorganic particles, 1176 g of talc having an average particle diameter of 12 µm and a thickness of 0.31 µm was mixed in 2807 g of deionized water to prepare a mixture solution having a weight ratio of the styrene butadiene resin to talc of 4/96 and a solid content concentration of 30% by weight. The mixture solution was dried by being sprayed in a dry atmosphere at a temperature of 70°C and at a humidity of 5%, and was collected into a vessel cooled to 10°C to obtain resin-coated scale-like inorganic particles (B). When this resin-coated scale-like inorganic particles (B) was applied to skin, softer feel and higher adhesiveness to skin was provided, as compared to talc alone applied to skin.

300 g of resin-coated scale-like inorganic particles (B) was added to and mixed with 1200 g of deionized water, and 200 g of spherical silica-titanium oxide composite particles having an average particle diameter of 1.2 µm (manufactured by CCIC: weight ratio of silica to titanium oxide; 85/15) was added and mixed with 1000 g of deionized water therewith, a dispersion solution with pH thereof adjusted to 9.0 by adding a dilute aqueous solution of sodium hydroxide was slowly added to the solution containing the particles (B) to continue mixing for one hour. The weight ratio of the spherical silica-titanium oxide composite particles and resin-coated scale-like inorganic particles (B) at that time was 40/60.

The resultant solution was filtered, dried and crushed to obtain scale-like composite particles (B-1) with spherical silica-titanium oxide composite particles adhered thereto.
When the scale-like composite particles (B-1) was applied to skin, oily feel and smooth and homogeneous applying characteristic was given, and a touch after the application provides oily feel and slippery characteristic.

### [Example 5]

As resin, 100 g of acrylic nitrile butadiene-based resin latex (manufactured by NIHON ZEON K.K.: glass transition temperature; -21°C, 100% modulus in the tensile test; 210 N/cm², resin particle diameter; 0.05 µm, solid content concentration; 41%) and, as scale-like inorganic particles, 1254 g of synthetic mica having an average particle diameter of 18 µm and a thickness of 0.38 µm was mixed in 5246 g of deionized water to prepare a mixture solution having a weight ratio of the butadiene resin to synthetic mica of 5/95 and a solid content concentration of 20% by weight. The mixture solution was dried by being sprayed in a dry atmosphere at a temperature of 70°C and at a humidity of 5%, and was collected into a vessel cooled to 10°C to obtain resin-coated scale-like inorganic particles (C). When this resin-coated scale-like inorganic particles (C) was applied to skin, softer feel and higher adhesiveness to skin was provided with being glazing suppressed, as compared to synthetic mica alone applied to skin.

300 g of resin-coated scale-like inorganic particles (C) was added to and mixed with 1200 g of deionized water, and 33 g of spherical alumina having an average particle diameter of 0.8 µm (manufactured by ADMATECHS K.K.: AO-502) was further mixed therewith in 165 g of deionized water. The weight ratio of the spherical alumina and resin-coated scale-like inorganic particles (C) at that time was 10/90.

The dispersion solution was spray-dried in the same way as Example 1 to obtain scale-like composite particles (C-1) with spherical alumina particles adhered thereto.
When the scale-like composite particles (C-1) was applied to skin, smooth and oily characteristic was given, and a touch after the application also kept the oily feel.

### [Comparative Example 1]

300 g of resin-coated scale-like inorganic particles (A) obtained in the same way as Example 1 was added to and mixed with 1200 g of deionized water, and 188 g of a spherical silica dispersion solution having an average particle diameter of 0.08 µm (manufactured by CCIC: Cataloid SI-80P, solid content concentration; 40%) was further mixed therewith. The weight ratio of the spherical silica and resin-coated scale-like inorganic particles (A) at that time was 20/80.
The dispersion solution was spray-dried in the same way as Example 1 to obtain scale-like composite particles (A-4). When the scale-like composite particles (A-4) was applied to skin, oily feel was low, and smoothness was insufficient.

### [Comparative Example 2]

300 g of resin-coated scale-like inorganic particles (B) obtained in the same way as Example 4 was added to and mixed with 1200 g of deionized water, and 200 g of spherical silica having an average particle diameter of 5 µm (manufactured by CCIC, SILICA MICROBEAD P-1500) was further added to and mixed with 1000 g of deionized water therewith. The weight ratio of the spherical silica and resin-coated scale-like inorganic particles (B) at that time was 40/60.
The dispersion solution was spray-dried in the same way as Example 1 to obtain scale-like composite particles (B-2). When the scale-like composite particles (B-2) was applied to skin, though smoothness was felt, there was not oily feel.

### [Comparative Example 3]

The scale-like inorganic particles (manufactured by CCIC: VELVET VEIL 640, weight ratio of silica particles to scale-like inorganic particles; 40:60) obtained by adhering spherical silica particles (average particle diameter; 0.55 µm) to mica employed in Example 1 were used, and, when the scale-like inorganic particles were applied to skin, though slippery characteristic was excellent and smoothness was felt, there was not oily feel, and a touch after the application provided lowered slippery characteristic and no oil feel.

### [Example 6]

Scale-like composite particles (A-1), (A-2), (A-3), (B-1) and (C-1) obtained in Example 1 to Example 5 respectively were used to prepare powder foundations each having the following composition.

| | | |
|---|---|---|
| (1) | scale-like composite particles | 20.0 |
| (2) | talc (silicone treated) | 43.2 |
| (3) | mica (silicone treated) | 5.0 |
| (4) | sericite (silicone treated) | 15.0 |
| (5) | pigment titanium oxide (silicone treated) | 5.0 |
| (6) | colcothar (silicone treated) | 0.7 |
| (7) | iron oxide (yellow) (silicone treated) | 1.5 |
| (8) | iron oxide (black) (silicone treated) | 0.4 |
| (9) | methyl polysiloxane | 5.0 |
| (10) | tri 2-ethylhexanoic acid glyceryl | 2.0 |
| (11) | liquid paraffin | 1.0 |
| (12) | diiso-polyglyceryl stearate | 1.0 |
| (13) | methyl paraoxybenzoate | 0.2 |

Firstly a mixture of (1) to (8) was prepared. (9) to (13) were mixed well while heated at 70°C, which was added to the mixture of (1) to (8) and mixed homogeneously. Then the resultant mixture was dried and crushed to make the particle size thereof uniform, and was compression molded. The obtained powder foundations (P₁), (P₂), (P₃), (P₄) and (P₅) were applied to skin, and subjected to sensory evaluation, the result of which is shown in Table 1.

The sensory evaluation was performed by 10 evaluators to compare feel (oily feel and slippery characteristic) when each of the powder foundations described above was applied on the evaluator' s forearm with a finger, and further to compare feel (oily feel and slippery characteristic) when the foundation-applied part was touched with a bare hand. The evaluation was determined on an ascending scale of 1 to 4 (4: Good, 3: Rather good, 2: Rather bad, and 1: Bad), and the scale values presented by the evaluators were averaged and rounded off, which are shown in Table 1.

### [Comparative Example 4]

Powder foundations (RP₁) and (RP₂) were prepared in the same way as Example 6 except that the scale-like composite particles (A-4) and (B-2) obtained in Comparative Examples 1 and 2 respectively were used instead of the scale-like composite particles in Example 6. The result of the sensory evaluation is shown in Table. 1.

### [Comparative Example 5]

Powder foundations (RP₃), (RP₄) and (RP₅) were prepared in the same way as Example 6 except that the scale-like composite particles (A), (B) and (C) obtained in Examples 1, 4 and 5 respectively were used instead of the scale-like composite particles in Example 6. The result of the sensory evaluation is shown in Table. 1.

### [Comparative Example 6]

Powder foundations (RP₆), (RP₇) and (RP₈) were prepared in the same way as Example 6 except that the scale-like composite particles (mica, talc and synthetic mica) employed in Examples 1, 4 and 5 respectively were used instead of the scale-like composite particles in Example 6. The result of the sensory evaluation is shown in Table. 1.

### [Comparative Example 7]

Powder foundations (RP₉) was prepared in the same way as Example 6 except that the scale-like composite particles with spherical silica particles adhered thereto obtained in Comparative Example 3 was used instead of the scale-like composite particles in Example 6. The result of the sensory evaluation is shown in Table. 1.

**Table 1**

| Powder foundation | Blended particle | In application | | After application | |
|---|---|---|---|---|---|
| | | Oily feel | Slippery characteristic | Oily feel | Slippery characteristic |
| Example 6 (P₁) | A-1 | 4 | 3 | 3 | 3 |
| Example 6 (P₂) | A-2 | 3 | 4 | 4 | 4 |
| Example 6 (P₃) | A-3 | 4 | 3 | 4 | 3 |
| Example 6 (P₄) | B-1 | 3 | 3 | 3 | 3 |
| Example 6 (P₅) | C-1 | 4 | 3 | 4 | 3 |
| Comparative Example 4 (RP₁) | A-4 | 2 | 1 | 2 | 1 |
| Comp. E. 4 (RP₂) | B-2 | 1 | 3 | 1 | 2 |
| Comp. E. 5 (RP₃) | A | 1 | 1 | 1 | 1 |
| Comp. E. 5 (RP₄) | B | 1 | 1 | 1 | 1 |
| Comp. E. 5 (RP₅) | C | 1 | 1 | 1 | 1 |
| Comp. E. 6 (RP₆) | mica | 1 | 2 | 1 | 1 |
| Comp. E. 6 (RP₇) | talk | 1 | 2 | 1 | 1 |
| Comp. E. 6 (RP₈) | synthetic mica | 2 | 2 | 1 | 1 |
| Comp. E. 7 (RP₉) | silica-coated mica | 2 | 4 | 1 | 2 |

### [Example 7]

Scale-like composite particles (A-1), (A-2), (A-3), (B-1) and (C-1) obtained in Example 1 to Example 5 respectively were used to prepare eye shadows each having the following composition.

| | | |
|---|---|---|
| (1) | scale-like composite particles | 30.0 |
| (2) | talc (silicone treated) | 10.0 |
| (3) | mica (silicone treated) | 34.51 |
| (4) | pearl pigment (silicone treated) | 10.0 |
| (5) | colcothar (silicone treated) | 0.08 |
| (6) | iron oxide (yellow) (silicone treated) | 0.2 |
| (7) | iron oxide (black) (silicone treated) | 0.01 |
| (8) | methyl polysiloxane | 7.5 |
| (9) | isotridecyl isononanoate | 5.0 |
| (10) | diiso-polyglyceryl stearate | 2.5 |
| (11) | methyl paraoxybenzoate | 0.2 |

Firstly a mixture of (1) to (7) was prepared. (8) to (11) were mixed well while heated at 70°C, which was added to the mixture of (1) to (7) and mixed homogeneously. Then the resultant mixture was dried and crushed to make the particle size thereof uniform, and was compression molded. The obtained eye shadows (I₁), (I₂), (I₃), (I₄) and (I₅) were, like the powder foundations described above, applied to skin, and were subjected to evaluation of the feel when applied on the forearm of the evaluators. The result is shown in Table 2.

### [Comparative Example 8]

Eye shadows (RI₁) and (RI₂) were prepared in the same way as Example 7 except that the scale-like composite particles (A-4) and (B-2) obtained in Comparative Examples 1 and 2 respectively were used instead of the scale-like composite particles in Example 7. The result of the sensory evaluation is shown in Table. 2.

**Table 2**

| Eye shadow | Blended particles | Oily feel | Slippery characteristic |
|---|---|---|---|
| Example 7 (I₁) | A-1 | 4 | 4 |
| Example 7 (I₂) | A-2 | 3 | 4 |
| Example 7 (I₃) | A-3 | 4 | 3 |
| Example 7 (I₄) | B-1 | 3 | 3 |
| Example 7 (I₅) | C-1 | 3 | 3 |
| Comparative Example 8 (RI₁) | A-4 | 1 | 2 |
| Comp. E. 8 (RI₂) | B-2 | 2 | 2 |

## Claims

1. Scale-like composite particles comprising scale-like inorganic particles coated with resin and spherical fine particles having an average particle diameter in a range from 0,1 to 3,0 µm adhered on a surface of said resin-coated particles wherein said spherical fine particles are made from inorganic oxide and adhesion occurs between said resin being positively charged when static electricity is generated and said spherical fine particles being negatively charged.

2. The scale-like composite particles according to Claim 1, wherein the fine particles adhere in a single layer.

3. The scale-like composite particles according to Claim 1, wherein a quantity of said spherical fine particles is in a range from 1/99 to 60/40 as expressed by a weight ratio of spherical fine particles versus resin-coated scale-like inorganic particles.

4. The scale-like composite particles according to Claim 1, wherein a quantity of resin coating said resin-coated scale-like inorganic particles is in a range from 0.1/99.9 to 20/80 as expressed by a weight ratio of resin versus scale-like inorganic particles.

5. The scale-like composite particles according to Claim 1, wherein an average particle diameter (Df) of said resin-coated scale-like inorganic particles is in a range from 1 to 50 µm, the average thickness (Tf) thereof is in a range from 0.01 to 1 µm, and the ratio (Df/Tf) of average diameter (Df) versus average thickness (Tf) is in a range from 10 to 300.

6. The scale-like composite particles according to Claim 1, wherein 100% modulus of said resin in a tensile test is in a range from 50 to 3000 N/cm².

7. The scale-like composite particles according to Claim 1, wherein said resin includes one or more selected from polyurethane, styrene-butadiene copolymer, acrylonitrile-butadiene copolymer, silicone-based elastomer and polyolefin-based elastomer.

8. Cosmetic preparations with the scale-like composite particles according to any of Claims 1 to 7 blended therein.

## Patentansprüche

1. Schuppenartige Kompositpartikel umfassend schuppenartige anorganische Partikel, die mit einem Harz überzogen sind, und kugelförmige feine Partikel mit einem durchschnittlichen Partikeldurchmesser in einem Bereich von 0,1 bis 3,0 µm, die auf einer Oberfläche der harzüberzogenen Partikel kleben, worin die kugelförmigen feinen Partikel aus einem anorganischen Oxid hergestellt sind und Adhäsion zwischen dem Harz, das positiv geladen ist, wenn eine statische Elektrizität erzeugt wird, und den kugelförmigen feinen Partikeln, die negativ geladen sind, auftritt.

2. Schuppenartige Kompositpartikel gemäß Anspruch 1, worin die feinen Partikel in einer einzigen Schicht anhaften.

3. Schuppenartige Kompositpartikel gemäß Anspruch 1, worin die Menge der kugelförmigen feinen Partikel in einem Bereich von 1/99 bis 60/40, ausgedrückt als Gewichtsverhältnis der kugelförmigen feinen Partikel zu den harzüberzogenen schuppenartigen anorganischen Partikeln, liegt.

4. Schuppenartige Kompositpartikel gemäß Anspruch 1, worin die Menge des Harzes, das die harzüberzogenen schuppenartigen anorganischen Partikel überzieht, in einem Bereich von 0,1/99,9 bis 20/80, ausgedrückt als Gewichtsverhältnis des Harzes zu den schuppenartigen anorganischen Partikeln, liegt.

5. Schuppenartige Kompositpartikel gemäß Anspruch 1, worin der durchschnittliche Partikeldurchmesser (Df) der harzüberzogenen schuppenartigen anorganischen Partikel in einem Bereich von 1 bis 50 µm liegt, die durchschnittliche Dicke (Tf) von diesen in einem Bereich von 0,01 bis 1 µm liegt und das Verhältnis (Df/Tf) des durchschnittlichen Durchmessers (Df) zu der durchschnittlichen Dicke (Tf) in einem Bereich von 10 bis 300 liegt.

6. Schuppenartige Kompositpartikel gemäß Anspruch 1, worin der 100%-Modul des Harzes in einer Zugfestigkeitsprüfung in einem Bereich von 50 bis 3000 N/cm² liegt.

7. Schuppenartige Kompositpartikel gemäß Anspruch 1, worin das Harz eines oder mehrere ausgewählt aus Polyurethan, Styrol-Buthadiencopolymer, Acrylonitril-Butadiencopolymer, Silicon-basiertes Elastomer und Polyolefin-basiertes Elastomer beinhaltet.

8. Kosmetische Zubereitungen mit schuppenartigen Kompositpartikeln gemäß einem der Ansprüche 1 bis 7, die darin untergemischt sind.

## Revendications

1. Particules composites lamellaires comprenant des particules minérales lamellaires revêtues de résine et des particules fines sphériques ayant un diamètre particulaire moyen dans une plage de 0,1 à 3,0 µm adhérant sur une surface desdites particules revêtues de résine, dans lesquelles lesdites particules fines sphériques sont réalisées à partir d'oxyde minéral et l'adhérence se produit entre ladite résine qui est chargée positivement lorsque de l'électricité statique est produite et lesdites particules fines sphériques étant chargées négativement.

2. Particules composites lamellaires selon la revendication 1, dans lesquelles les particules fines adhèrent en une couche unique.

3. Particules composites lamellaires selon la revendication 1, dans lesquelles une quantité desdites particules fines sphériques se situe dans la plage de 1/99 à 60/40, telle qu'exprimée par un rapport pondéral des particules fines sphériques par rapport aux particules minérales lamellaires revêtues de résine.

4. Particules composites lamellaires selon la revendication 1, dans lesquelles une quantité de résine revêtant lesdites particules minérales lamellaires revêtues de résine se situe dans une plage allant de 0,1/99,9 à 20/80, tel qu'exprimée par un rapport pondéral de résine par rapport aux particules minérales lamellaires.

5. Particules composites lamellaires selon la revendication 1, dans lesquelles un diamètre particulaire moyen (Df) desdites particules inorganiques lamellaires revêtues de résine se situe dans une plage de 1 à 50 µm, l'épaisseur moyenne (Tf) de celles-ci se situant dans une plage de 0,01 à 1 µm, et le rapport (Df/Tf) de diamètre moyen (Df) par rapport à l'épaisseur moyenne (Tf) se situe dans une plage de 10 à 300.

6. Particules composites lamellaires selon la revendication 1, dans lesquelles un module 100 % de ladite résine dans un test de résistance à la traction se situe dans une plage de 50 à 3000 N/cm².

7. Particules composites lamellaires selon la revendication 1, dans lesquelles ladite résine comprend un ou plusieurs éléments choisis parmi le polyuréthane, le copolymère styrène-butadiène, le copolymère acrylonitrile-butadiène, l'élastomère à base de silicone et l'élastomère à base de polyoléfine.

8. Préparations cosmétiques avec les particules composites lamellaires selon l'une quelconque des revendications 1 à 7 mélangées dans celles-ci.
